# EUROPEAN PATENT APPLICATION

(11) **EP 4 663 790 A1**
(43) Date of publication of application: **17.12.2025**
(21) Application number: 25182256.5
(22) Date of filing: 12.06.2025
(51) Int. Cl.: C22C 23/04, A61L 27/04, A61L 27/58, B21C 1/00, B21C 23/00, C22F 1/06

(54) **METHOD OF PRODUCING A MICROALLOYED MAGNESIUM ALLOY**

(30) Priority: 14.06.2024 DE 102024116789
(71) Applicant: MgSANA Corp., Mobile AL 36602 (US)
(72) Inventor: Prof. Dr. ELIEZER, Amir, 8496500 Omer (IL)
(74) Representative: Patentship Patentanwaltsgesellschaft mbH

(57) **Abstract**

The disclosure relates to a method of producing a microalloyed magnesium alloy, wherein the magnesium alloy comprises 1-2 weight% Zn, 0.5 - 1 weight% Mn and 0.2 - 0.8 weight% Ca, wherein a billet is heated to a temperature between 220 to 340 °C and wherein said billet is drawn to a rod in the heated state.

## Description

### Field of the Invention

The invention relates to a microalloyed magnesium alloy and a method for production thereof. In particular, the invention relates to a bioresorbable magnesium alloy for medical applications.

### Background of the Invention

Document WO 2019/002277 A1 shows a bioresorbable magnesium alloy, which comprises calcium, manganese and zinc.

### Object of the Invention

It is an object of the invention to provide a magnesium alloy, in particular for medical applications, with improved mechanical properties.

### Summery of the Invention

The object of the invention is achieved by a method for producing a microalloyed magnesium alloy according to claim 1.

Preferred embodiments of the inventions are subject of the dependent claims, the description and the drawings.

The invention relates to method of producing a microalloyed magnesium alloy. The magnesium alloy comprises 1-2 weight% Zn, 0.5 - 1 weight% Mn and 0.2 - 0.8 weight% Ca. Preferably, the alloy comprises 97 weight% Mg, except of Mn, Ca, and Mg, not any other alloying elements. The magnesium alloy is free of any rare earth elements.

According to the invention, a billet is heated to a temperature between 220 to 340 °C and then drawn to a rod in the heated state. The drawing process can be performed by using an extrusion machine.

According to a preferred embodiment, the billet is heated to a temperature of 400 to 500 °C.

Then, the billed is cooled down, preferably to room temperature.

Then, the billet to the temperature of 300 to 400 °C and drawn to the rod in the heated state of 220 to 340 °C.

Preferably, the billet is drawn with a temperature of 240 to 310 °C.

Preferably, the billet is heated to a temperature of 400 to 500 °C for 2 to 8 hours.

According to an embodiment, the diameter of the billet is reduced by drawing the billet in two steps. This results in reducing the grain size to a preferred range. In particular, a grain size of 0.7 to 2.4. µm result is an improved mechanical strength and in an increased corrosion resistance also.

According to an embodiment, the diameter of the billet is reduced in a first drawing step to a 0.1 to 0.5 smaller diameter. The diameter of the billet can be reduced in a second drawing step to a 0.1 to 0.3 smaller diameter.

At least the second drawing step is performed at the temperature of 220 to 340 °C, preferably at 240 to 310 °C.

In further the invention relates to a microalloyed magnesium alloy, in particular being produced with a method as described above.

The magnesium alloy comprises 1-2 weight% Zn, 0.5 - 1 weight% Mn and 0.2 - 0.8 weight% Ca.

The magnesium alloy has grain size between 0.7 and 2.4 µm, a tensile yield strength (TYS) of more than 200 MPa and an elongation at fracture of more than 18 %.

Preferably, the magnesium alloy has a TYS of 250 - 380 MPa.

Preferably, the magnesium alloy has a TYS of more than 300 MPa.

Preferably, the magnesium alloy has an elongation at fracture of 18 to 35 %.

Preferably, the magnesium alloy has an ultimate tensile strength of more than 250 MPa, in particular of 250 MPa to 390 MPa.

Elongation at fracture (A), TYS and UTS are determined according to DIN EN ISO 6892-1 - 2020-06. An MTS universal testing machine 810 with tactile strain gauge can be used.

The invention further relates to a medical implant which comprises, in particular which consists of the microalloyed magnesium alloy.

The alloy is biodegradable. According to an embodiment, the alloy comprises a coating, particularly a calcium phosphate coating.

### Brief Description of the Drawings

Fig. 1 and 2 are microscopic images of a magnesium alloy according to an embodiment of the invention.
Fig. 3 is a flow chart of a process for producing an implant according to an embodiment of the invention.
Fig. 4 and Fig. 5 are Nyquist plots of alloys which had been drawn at different temperatures.

### Detailed description of the Drawings

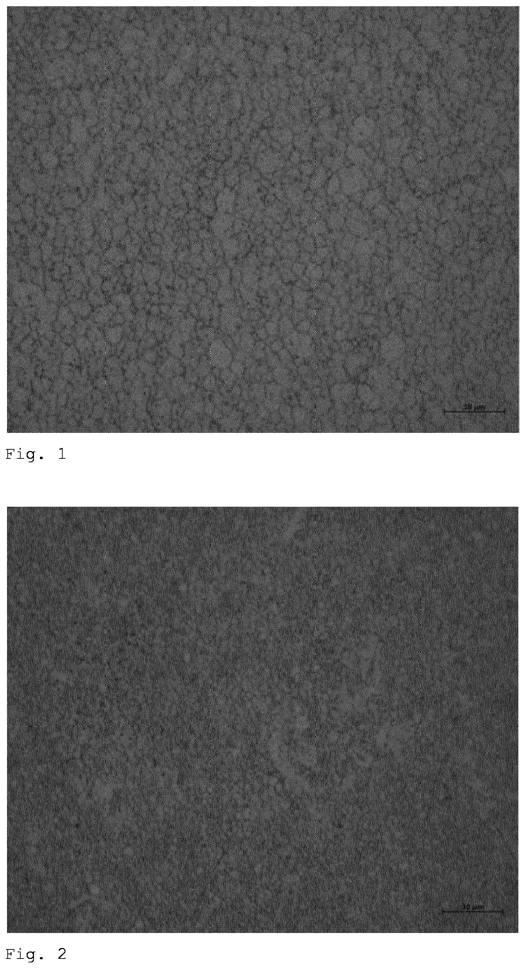

Fig. 1 and 2 are microscopic images of a magnesium alloy according to an embodiment of the invention.

For the alloy according to Fig. 1, the billet had been drawn at a temperature of 310 °C. The drawn alloy has a TYS of approx. 250 MPa, an UTS of approx. 290 MPa and an elongation A of approx. 25 %. The grain size is 1.7 µm.

For the alloy according to Fig. 2, the billet had been drawn at a temperature of 240 °C. The drawn alloy has a TYS of approx. 360 MPa, an UTS of approx. 350 MPa and an elongation A of approx. 15 %. The grain size is 1.0 µm.

Hence, a higher temperature results in slightly poorer mechanical strength but results in a higher elongation.

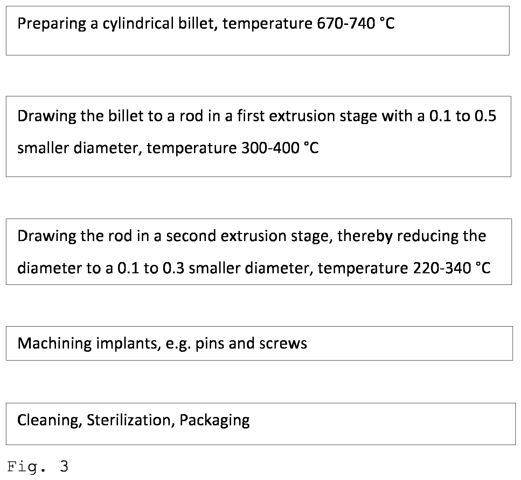

Fig. 3 shoes a process for producing an implant according to an embodiment of the invention.

First, a cylindrical billet is casted, the billet temperature is 670-740 °C.

Then, the billet is drawn to a rod in a first extrusion stage with a 0.1 to 0.5 smaller diameter as the billet, the temperature is 300-400 °C.

Then, the rod is drawn in a second extrusion stage, thereby reducing the diameter to a 0.1 to 0.3 smaller diameter, the temperature is 220-340 °C.

Then, the rod is machined to an implant, e.g. to a pins or screw.

Finally, the implant is cleaned, sterilized and packaged.

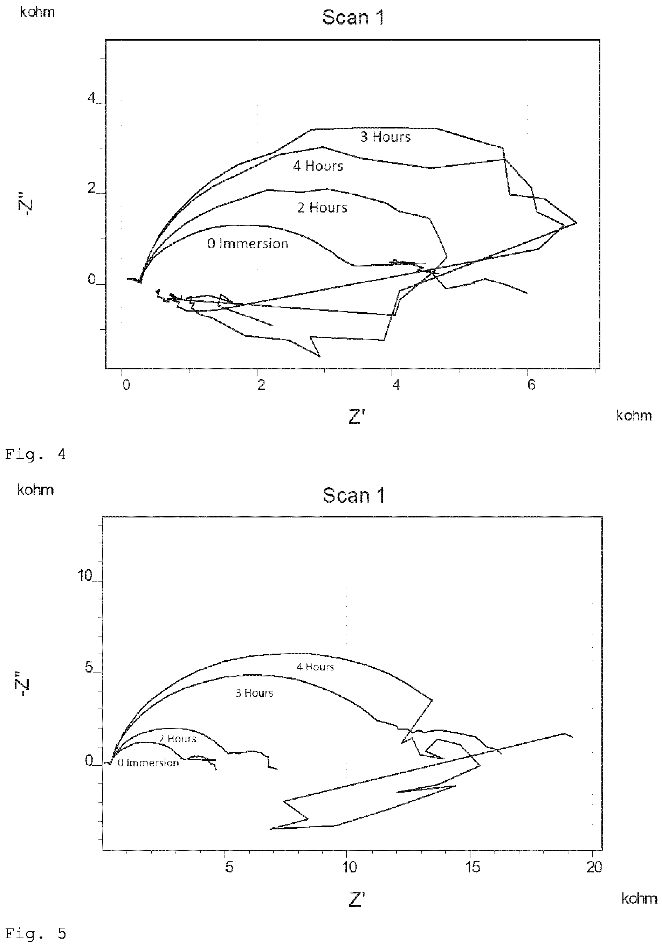

Fig. 4 is a Nyquist plot of an Mg alloy which hand been drawn at 240 °C and which has a grain size of 0.9 µm. The alloy had been immersed in a saline solution for 0, 2, 3 and 4 hours.

Fig. 5 is a Nyquist plot of an Mg alloy which hand been drawn at 310 °C and which has a grain size of 1.5 µm.

The Nyquist impedance can used to determine the corrosion resistance of the alloy.

Regarding the impedance, the alloy according to Fig. 4 has a high corrosion resistance and has a high mechanical strength.

Overall, at 0,1,3,4 hours of exposure the vertical Nyquist axis is up to 4 Z'' kOhm and the horizontal axis is up to 8 Z' kOhm.

The alloy according to Fig. 5 has at 0,1,3,4 hours of exposure, a vertical Nyquist axis up to 7 Z'' kOhm and a horizontal up to 18 Z' kOhm.

The Nyquist plot shows the relationship between grain size, drawing temperature and corrosion resistance.

In comparison to Fig. 4, the alloy according to Fig. 5 was drawn with a higher process temperature which results in a larger grain size which results a higher impedance which shows a higher corrosion resistance.

The lower process temperature results in a smaller grain size and better mechanical properties. A smaller grain size results, due to Gibbs Energy and higher amount of grain boundaries, in a smaller corrosion resistance.

## Claims

1. Method of producing a microalloyed magnesium alloy,
wherein the magnesium alloy comprises 1-2 weight% Zn, 0.5 - 1 weight% Mn and 0.2 - 0.8 weight% Ca, wherein a billet is heated to a temperature between 220 to 340 °C and wherein said billet is drawn to a rod in the heated state.

2. The method according to claim 1, comprising the steps:
- heating the billet to a temperature of 400 to 500 °C;
- cooling down the heated billet;
- heating the billet to the temperature of 240 to 340 °C;
- drawing the billet to the rod in the heated state of 220 to 340 °C.

3. The method according to any of the preceding claims,
wherein the billet is drawn with a temperature of 240 to 310 °C.

4. The method according to any of the preceding claims,
wherein the billet is heated to a temperature of 400 to 600 °C for 2 to 8 hours.

5. The method according to any of the preceding claims,
wherein the diameter of the billet is reduced by drawing the billet in two steps.

6. The method according to the preceding claim, wherein the diameter of the billet is reduced in a first drawing step to a 0.1 to 0.5 smaller diameter and/or wherein the diameter of the billet is reduced in a second drawing step to a 0.1 to 0.3 smaller diameter.

7. Microalloyed magnesium alloy, in particular being produced with a method according to any of the preceding claims, wherein the magnesium alloy comprises 1-2 weight% Zn, 0.5
- 1 weight% Mn and 0.2 - 0.8 weight% Ca, wherein said magnesium alloy has grain size between 0.7 and 2.4 µm, a tensile yield strength (TYS) of more than 200 MPa and an elongation at fracture of more than 10 %, preferably 18 %.

8. Microalloyed magnesium alloy according to the preceding claim, wherein the magnesium alloy has a TYS of 250 - 390 MPa.

9. Microalloyed magnesium alloy according to any of the preceding claims, wherein the magnesium alloy has a TYS of more than 300 MPa.

10. Microalloyed magnesium alloy according to any of the preceding claims, wherein the magnesium alloy has an elongation at fracture of 18 to 35 %.

11. Microalloyed magnesium alloy according to any of the preceding claims, wherein the magnesium alloy has an ultimate tensile strength of more than 250 MPa, in particular of 250 MPa to 390 MPa.

12. Medical implant, comprising a microalloyed magnesium alloy according to any of the preceding claims.
